# EUROPEAN PATENT APPLICATION

(11) **EP 3 657 173 A1**
(43) Date of publication of application: **27.05.2020**
(21) Application number: 18382842.5
(22) Date of filing: 22.11.2018
(51) Int. Cl.: G01N 33/574

(54) **BIOMARKERS OF PROSTATE CANCER AGGRESSIVENESS**

(71) Applicant: Institut d'Investigació Sanitària Pere Virgili, 43003 Tarragona (ES)
(72) Inventor: RODRÍGUEZ CHACÓN, Matilde, 43005 Tarragona (ES); RUIZ PLAZAS, Xavier, 43005 Tarragona (ES); SEGARRA TOMÁS, Josep, 43005 Tarragona (ES); GARCÍA FONTGIVELL, Joan Francesc, 43005 Tarragona (ES); LOZANO BARTOLOMÉ, Javier, 43005 Tarragona (ES); MARTÍNEZ GONZÁLEZ, Salomé, 43005 Tarragona (ES)
(74) Representative: Carvajal y Urquijo, Isabel

(57) **Abstract**

The present invention is directed to an *in vitro* method for the differential diagnosis of high risk or intermediate risk prostate cancer versus low risk prostate cancer, or for determining the likelihood that a patient with prostate cancer develops a high risk or intermediate risk prostate cancer. The invention also provides a method for selecting a therapy for a patient suffering from prostate cancer, or for selecting a patient with prostate cancer for a treatment selected from surgery, radiotherapy or a combination thereof. Additionally, the invention provides a kit comprising reagents for determining the levels of sTWEAK protein and at least an additional reagent for the quantification of the level of a biomarker selected from the group consisting of total PSA, FN14, preferably sFN14, sCD163 and glucose, and the uses of this kit.

## Description

### FIELD OF THE INVENTION

The invention relates to the field of biomarkers of the aggressiveness of prostate cancer, in particular, biomarkers for the differential diagnosis between high or intermediate risk prostate cancer and low risk prostate cancer.

### BACKGROUND OF THE INVENTION

Prostate cancer is one of the most common types of cancers in men. Usually prostate cancer grows slowly and is initially confined to the prostate gland however, while some types of prostate cancer grow slowly and may need minimal or even no treatment, other types are aggressive and can spread quickly. Given that the therapeutic approaches vary greatly depending on the aggressiveness of the tumor, distinguishing between low and high-grade prostate cancer is of great importance. However, biopsy, besides being invasive, may underestimate the actual grade of cancer. On the other hand, many biomarkers have been postulated as prognostic markers for prostate cancer to date, such as B7-H3 (CD276), Ki-6, EPCA, LAT1 (CD98), BRCA1/BRCA, MME, proteins in urine such as Annexine A3 (ANXA), MMPs, delta-catenin, hepatocyte growth factor (c-met), thymosine, metabolites such as sarcosin, genetic alterations such as GSTP1, RASSF2, HIST1H4K or TFAP2E hypermethylation, or markers based on the RNA resulting from the transcription of the TMPRSS2-ERG or PCA3 fusion gene. Perhaps the most studied biomarker in prostate cancer has been the prostate specific antigen (PSA).

However, preanalytical factors such as the variation between methods of collection, handling and storage of samples, the inconsistent number of patients between studies and the heterogeneity of the populations studied have limited the possible validation of the results obtained.

Therefore, there is a need for new reliable and non-invasive methods for classifying the aggressiveness of prostate cancer.

### BRIEF DESCRIPTION OF THE INVENTION

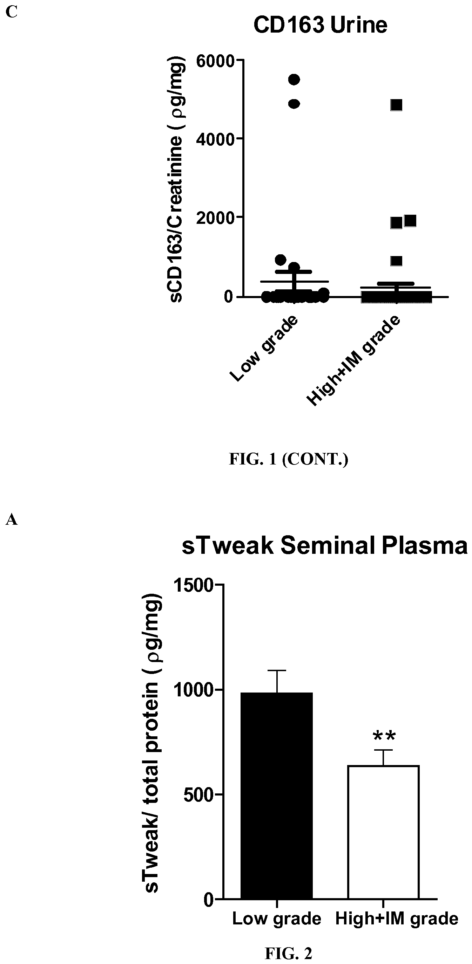

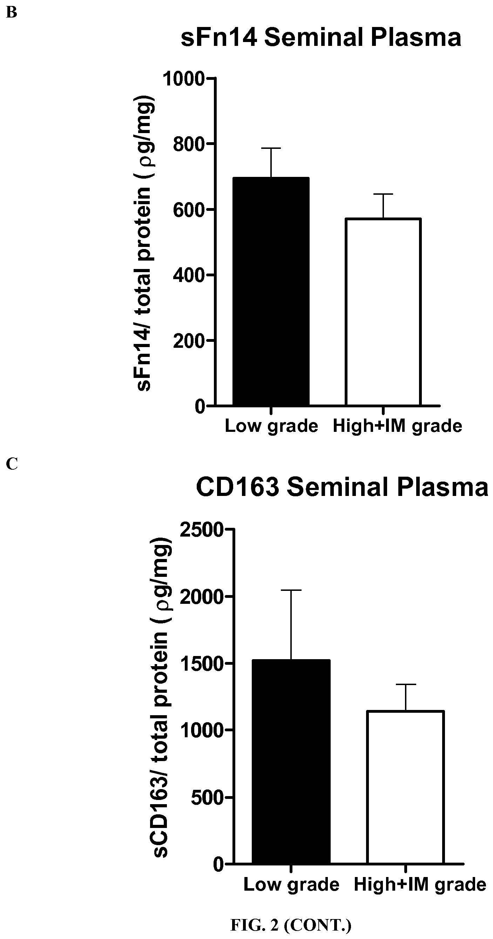

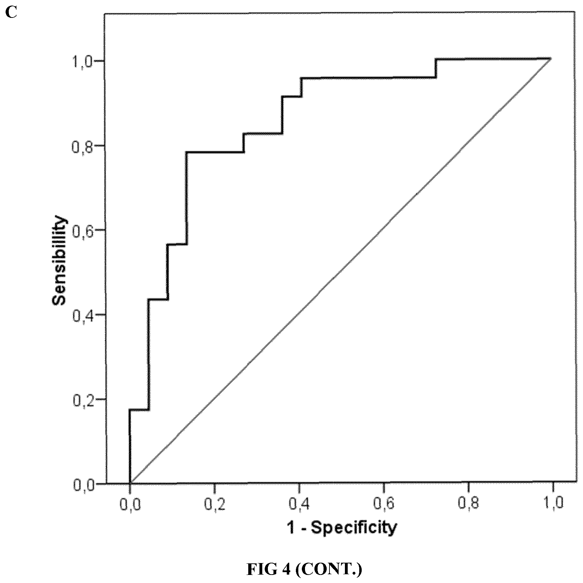

The authors of the present invention have surprisingly found that soluble TWEAK (sTWEAK) levels are significantly reduced in seminal plasma from patients with more aggressive prostate cancer (Figure 2). Additionally, the inventors have shown that a multivariate logistic regression model composed of the variants total PSA serum levels, sTWEAK, sFn14, and sCD163 seminal levels and plasma glucose levels can correctly classify 80% of the patients, with a sensitivity of 82.6% and a specificity of 72.7% (Figure 4C).

Therefore, in a first aspect, the invention relates to an *in vitro* method for the differential diagnosis of high risk or intermediate risk prostate cancer from low risk prostate cancer, or for determining the likelihood that a patient with prostate cancer develops a high risk or intermediate risk prostate cancer, comprising:
(a) determining the level of soluble tumor necrosis factor ligand superfamily member 12 (sTWEAK) protein in a sample of semen from the patient and
(b) comparing said level with a reference value
wherein
- a decreased level of sTWEAK compared to the reference value is indicative that the patient suffers from a high risk or intermediate risk prostate cancer, or that there is a high likelihood that the patient develops a high risk or intermediate risk prostate cancer, or, alternatively,
- an equal or increased level of sTWEAK compared to the reference value is indicative that the patient suffers from low risk prostate cancer, or that there is a low likelihood that the patient develops a high risk or intermediate risk prostate cancer.

In a second aspect, the invention relates to a method for selecting a therapy for a patient suffering from prostate cancer, comprising:
(a) determining the level of sTWEAK in a sample of semen from the patient and
(b) comparing said level with a reference value
wherein
- if a decreased level of sTWEAK compared to the reference value is detected, a therapy selected from the group consisting of prostate surgery, radiotherapy and a combination thereof is selected or, alternatively,
- if an equal or increased level of sTWEAK compared to the reference value is detected, an alternative therapy is selected.

In a third aspect, the invention relates to a method for selecting a patient suffering from prostate cancer for a treatment selected from the group consisting of prostate surgery, radiotherapy and a combination thereof, comprising:
(a) determining the level of sTWEAK in a sample of semen from the patient and
(b) comparing said level with a reference value
wherein the patient is selected for said treatment if a decreased level of sTWEAK compared to the reference value is detected.

In a fourth aspect, the invention relates to a kit comprising a reagent specific for determining the level of sTWEAK protein and at least an additional reagent specific for determining the level of a biomarker selected from the group consisting of total PSA, FN14, particularly sFN14, sCD163 and glucose.

In a fifth aspect, the invention relates to the use of the kit of the fourth aspect for:
(a) the differential diagnosis of high risk or intermediate risk prostate cancer from low risk prostate cancer; or
(b) for determining the likelihood that a patient with prostate cancer develops a high risk or intermediate risk prostate cancer; or
(c) for selecting a therapy for a patient suffering from prostate cancer; or
(d) for selecting a patient suffering from prostate cancer for a treatment selected from the group consisting of prostate surgery, radiotherapy and a combination thereof.

### BRIEF DESCRIPTION OF THE DRAWINGS

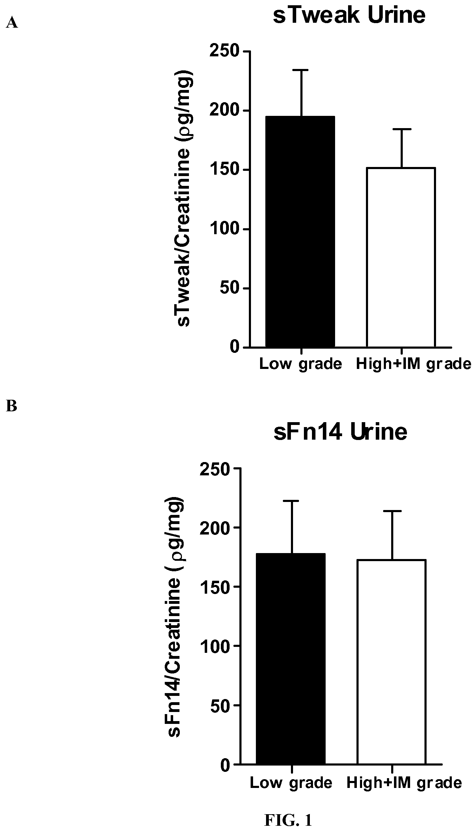

**Figure 1****. sTWEAK, sFn14 and sCD163 levels in human urine samples.** Plots represent concentration of sTWEAK, sFn14 and sCD163 in human urine classified by D'Amico Risk Classification for Prostate Cancer. Low grade and high plus medium grade were analysed together. Error bars represent the standard deviation.
**Figure 2****. sTWEAK, sFn14 and sCD163 levels in seminal plasma samples.** Plots represent concentration of sTWEAK, **sFn14** and sCD163 in human urine classified by D'Amico Risk Classification for Prostate Cancer. Low grade and high plus medium grade were analysed together. Error bars represent the standard deviation. Double asterisk indicate p<0.001.

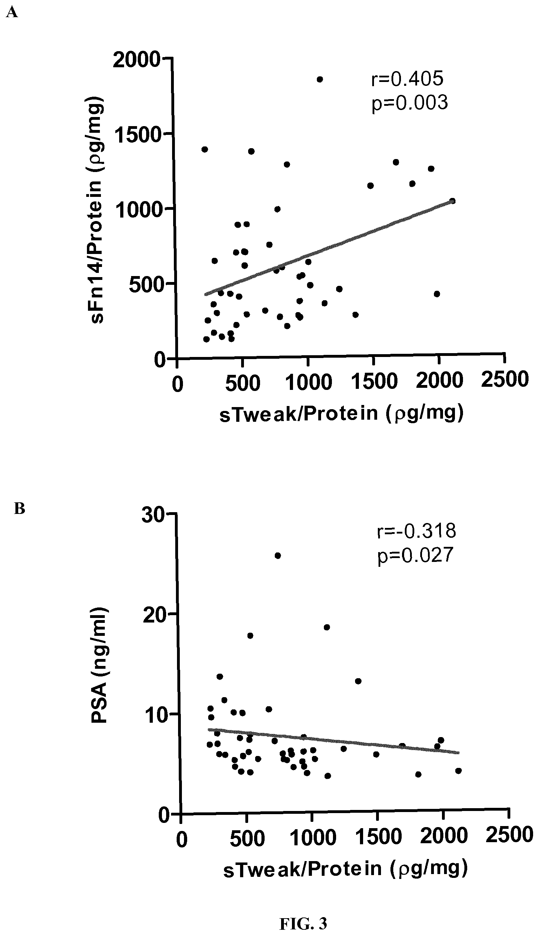

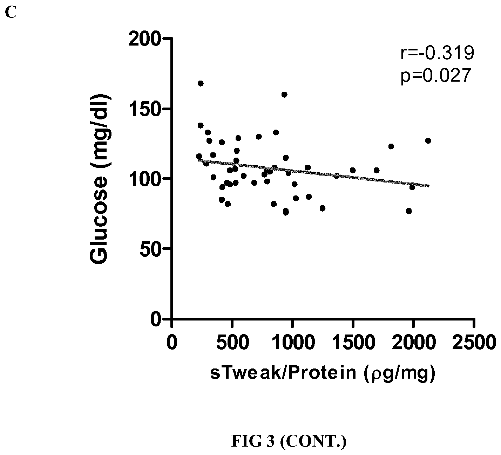

**Figure 3****. Dispersion graphs of Spearman correlation data: A)** sTWEAK pg/mg total protein seminal plasma vs sFn14 pg/mg of total protein in seminal plasma. **B)** total PSA serum levels ng/mL vs sFn14 pg/mg of total protein in seminal plasma. **C)** Glucose serum levels mg/dL vs sTWEAK pg/mg of total protein in seminal plasma

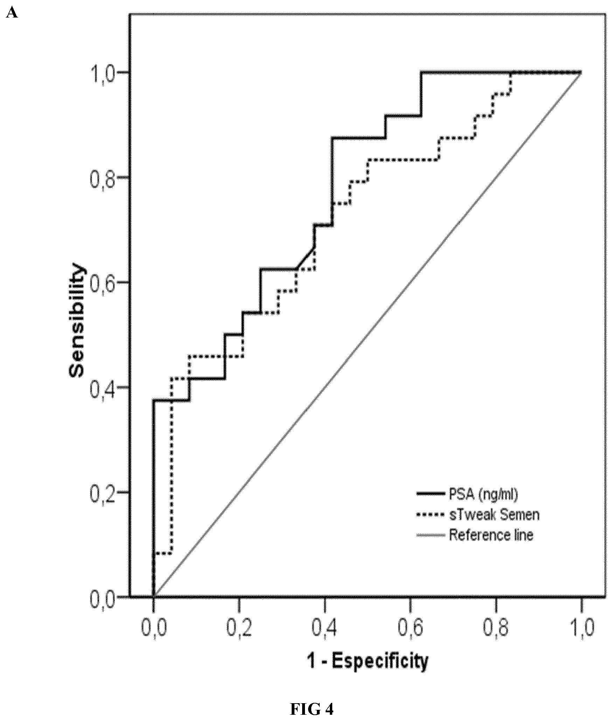

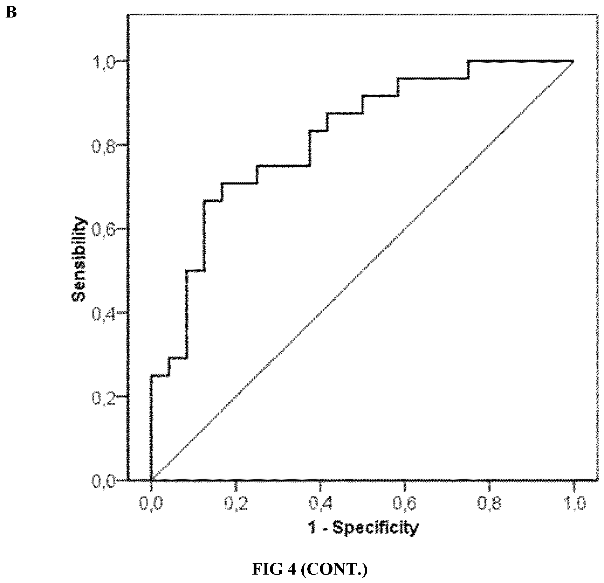

**Figure 4****. Receiver operating characteristic curves. A)** for total PSA plasma levels and sTWEAK levels in seminal plasma individually. **B)** for total PSA levels and sTWEAK in seminal plasma combined. **C)** Combined levels of 5 biomarkers: total PSA in plasma sTWEAK, sFn14 and sCD163 in seminal plasma and plasma glucose levels.

### DETAILED DESCRIPTION OF THE INVENTION

### Method of differential diagnosis of prostate cancer

In a first aspect, the invention relates to an *in vitro* method for the differential diagnosis of high risk or intermediate risk prostate cancer from low risk prostate cancer, or for determining the likelihood that a patient with prostate cancer develops a high risk or intermediate risk prostate cancer, hereinafter first method of the invention, comprising:
(a) determining the level of soluble tumor necrosis factor ligand superfamily member 12 (sTWEAK) protein in a sample of semen from the patient and
(b) comparing said level with a reference value
wherein
- a decreased level of sTWEAK compared to the reference value is indicative that the patient suffers from a high risk or intermediate risk prostate cancer, or that there is a high likelihood that the patient develops a high risk or intermediate risk prostate cancer, or, alternatively,
- an equal or increased level of sTWEAK compared to the reference value is indicative that the patient suffers from low risk prostate cancer, or that there is a low likelihood that the patient develops a high risk or intermediate risk prostate cancer.

The term "*in vitro*", as used herein, refers to the fact that the method is not carried out on the body of a human or animal subject, but rather on cells or fluids isolated from said subject or in a test tube.

The term "differential diagnosis", as used herein, means a diagnostic method used to identify the presence of an entity where multiple alternatives are possible (the process may be termed differential diagnostic procedure), and may also refer to any of the included candidate alternatives (which may also be termed candidate condition). This method is essentially a process of elimination or at least of obtaining information that shrinks the "probabilities" of candidate conditions to negligible levels. The "probabilities" at issue are epistemic rather than ontological in that they are imaginative parameters in the mind of the diagnostician (or, for computerized or computer-assisted diagnosis, the software of the system), while in reality the target (such as a patient) either has a condition or not with an actual probability of either 0 or 100%. As used herein, the term "differential diagnosis" includes discriminating between two conditions, namely: (a) high or intermediate risk prostate cancer and (b) low risk prostate cancer.

This diagnosis, as it is understood by a person skilled in the art, does not claim to be correct in 100% of the analyzed samples. However, it requires that a statistically significant amount of the analyzed samples are classified correctly. The amount that is statistically significant can be established by a person skilled in the art by means of using different statistical tools; illustrative, non-limiting examples of said statistical tools include determining confidence intervals, determining the p-value, the Student's t-test or Fisher's discriminating functions, etc. Preferred confidence intervals are at least 90%, at least 97%, at least 98%, at least 99%. The p-values are, preferably less than 0.1, less than 0.05, less than 0.01, less than 0.005 or less than 0.0001. The teachings of the present invention preferably allow correctly diagnosing in at least 60%, in at least 70%, in at least 80%, or in at least 90% of the subjects of a group or population analyzed.

The term "prostate cancer", as used herein, refers to any malignant proliferative disorder of prostate cells.

According to the original Gleason score (Gleason et al., "Histologic grading and staging of prostatic carcinoma", Tannenbaum M. ed. Urologic Pathology, 1977: 171-187), prostate cancer can be classified according to their aggressiveness as:
- low aggressive tumours, those having a Gleason score of 5 or below;
- intermediate aggressive tumours, those having a Gleason score of 6; and
- highly aggressive tumours, those having a Gleason score of 7 or higher, until 10, with the more aggressive forms of prostate cancer being those which have scores of 8, 9, or 10.

The original Gleason score has been updated by a new grading system that has been accepted by the 2016 World Health Organization (WHO), described by Epstein et al., Eur Urol. 2016, 69(3): 428-35.

The term "prostate cancer" encompasses prostate tumors of any type of aggressiveness.

Patients with prostate cancer can be stratified into the three risk groups following the criteria of D'amico (V. D. Amico, et al., "Biochemical Outcome After Radical Prostatectomy, External Beam Radiation Therapy, or Interstitial Radiation Therapy for Clinically Localized Prostate Cancer," JAMA. 1998;280(11):969-974.). These criteria include prostate specific antigen (PSA) serum levels described by Stamey et al., N. Engl. J. Med., 1987, 317(15): 909-16, Gleason score and stage using the 1992 American Joint Commission on Cancer (AJCC) staging system (Beahrs et al., "Manual for staging of cancer", 4th ed. Philadelphia: J. B. Lippincott; 1992: 181-186):
- High risk: prostate specific antigen (PSA) level in serum > 20 ng/mL and Gleason score of 8-10 T2c-T3.
- Intermediate risk: PSA 10-20 ng / mL, Gleason score 7, T2b.
- Low risk: PSA <10 ng/mL, Gleason score 6, T1c, T2a.

The term "prostate cancer" encompasses prostate tumors of any of the above risk groups.

The terms "high risk prostate cancer", "intermediate risk prostate cancer" and "low risk prostate cancer" refer to the risk groups defined according to the D'Amico criteria explained above.

The term "determining the likelihood", as used herein, refers to determining the probability of a particular event. In the context of the first method of the invention, determining the likelihood that a patient with prostate cancer develops high risk or intermediate risk prostate cancer refers to determining whether said patient has a high likelihood of developing high risk or intermediate risk prostate cancer.

The term "patient" or "subject", as used herein, relates to any male animal, preferably a mammal and includes, but is not limited to, domestic and farm animals, primates, and humans, for example, human beings, non-human primates, cows, horses, pigs, sheep, goats, dogs, cats, or rodents like rats and mice. In a preferred embodiment, the subject is a human being of any age or race. In a particular embodiment, the patient suffers from prostate cancer, that is, he has been diagnosed as suffering from prostate cancer. A patient is diagnosed of suffering from prostate cancer if he fulfils the histopathologic diagnostic criteria (see for example Gleason and Mellinger, J Urol. 1974;111:58-64 and Epstein et al., Am J Surg Pathol. 2016, 40(2):244-52).

The first method of the invention comprises a first step of determining the level of soluble tumor necrosis factor ligand superfamily member 12 or TWEAK protein in a sample of semen from the patient.

The term "tumor necrosis factor ligand superfamily member 12" or "TWEAK" or "TNF-related weak inducer of apoptosis", as used herein, refers to a protein that in humans is encoded by the TNFSF12 gene. TWEAK is a ligand for the FN14/TWEAKR receptor and has overlapping functions with TNF, but with a much wider distribution. In humans, TWEAK corresponds to the protein defined under accession number O43508 in UniProtKB/Swiss-Prot (Entry version 163 of 12 Sep 2018, Sequence version 1 of 1 June 1998). TWEAK exits in two forms: a membrane associated form, that in humans is 249 amino acids length, and a soluble form, which originates from the proteolytic processing of the membrane form, and that in humans is 156 amino acid long. Therefore, the term "soluble TWEAK" or "sTWEAK", as used herein, refers to the processed from of TWEAK, which is not membrane associated.

The term "sTWEAK level", as used herein, refers to the amount of the sTWEAK protein that is found in a particular sample.

The level of a protein can be determined by any method known in the art suitable for the determination and quantification of a protein in a sample. By way of a non-limiting illustration, the level of a protein can be determined by means of a technique which comprises the use of antibodies with the capacity for binding specifically to the assayed protein (or to fragments thereof containing the antigenic determinants) and subsequent quantification of the resulting antigen-antibody complexes, or alternatively by means of a technique which does not comprise the use of antibodies such as, for example, by techniques based on mass spectroscopy. The antibodies can be monoclonal, polyclonal or fragments thereof, Fv, Fab, Fab' and F(ab')2, scFv, diabodies, triabodies, tetrabodies and humanized antibodies. Similarly, the antibodies may be labeled. Illustrative, but non-exclusive, examples of markers that can be herein used include radioactive isotopes, enzymes, fluorophores, chemoluminescent reagents, enzyme cofactors or substrates, enzyme inhibitors, particles, or dyes. There is a wide variety of known tests that can be used according to the present invention, such as combined application of non-labeled antibodies (primary antibodies) and labeled antibodies (secondary antibodies), Western blot or immunoblot, ELISA (enzyme-linked immunosorbent assay), RIA (radioimmunoassay), competitive EIA (enzyme immunoassay), DAS-ELISA (double antibody sandwich ELISA), two-dimensional gel electrophoresis, capillary electrophoresis, immunocytochemical and immunohistochemical techniques, immunoturbidimetry, immunofluorescence, techniques based on the use of biochips or protein microarrays including specific antibodies or assays based on the colloidal precipitation in formats such as reagent strips and assays based on antibody-linked quantum dots. Other forms of detecting and quantifying proteins include, for instance, affinity chromatography techniques or ligand-binding assays.

The level of sTWEAK can be determined by submitting the sample to a step to remove intact cells, cell debris and membrane fragments, and applying the above protein determination techniques to the supernatant. For example, in a particular embodiment, a sample of semen is centrifuged at 2000g for approximately 15 minutes obtaining a supernatant, which is called seminal plasma, where the level of sTWEAK is determined.

In a particular embodiment, the level of sTWEAK is determined by ELISA.

The term "sample of semen", as used herein, refers to any material containing sperm, whether processed or unprocessed, and includes ejaculates, electroejaculates, sperm isolated from testes or epididymis extended semen, sperm prepared by swim-up procedures, and sperm prepared by percoll gradient centrifugation. Depending on the subject's condition, the semen may or may not contain spermatozoa.

In a particular embodiment, the sample of semen is a sample of seminal plasma. As used herein, the term "seminal plasma" or "seminal fluid" generally refers to the liquid supernatant, after sedimentation or centrifugation of semen, which has been contributed by the testis and the various sex glands (e.g. the seminal vesicles, the prostate and the bulbourethral glands).

The second step of the first method of the invention comprises comparing the level of sTWEAK with a reference value.

The term "reference value", as used herein, relates to a predetermined criteria used as a reference for evaluating the values or data obtained from the samples collected from a subject. The reference value or reference level can be an absolute value, a relative value, a value that has an upper or a lower limit, a range of values, an average value, a median value, a mean value, or a value as compared to a particular control or baseline value. A reference value can be based on a large number of samples, such as from population of subjects of the chronological age matched group, or based on a pool of samples.

In a particular embodiment, the reference value is the level of sTWEAK in one or more subjects that are known to suffer from low risk prostate cancer. In this particular embodiment, a decreased level of sTWEAK compared to its reference value is indicative that the patient suffers from a high risk or intermediate risk prostate cancer or that there is a high likelihood that the patient develops high risk or intermediate risk prostate cancer; and an equal or increased level of sTWEAK compared to its reference value is indicative that the patient suffers from low risk prostate cancer, or that there is a low likelihood that the patient develops a high risk or intermediate risk prostate cancer.

In another particular embodiment, the reference value is the level of sTWEAK in one or more subjects who suffer from high or intermediate risk prostate cancer. In this particular embodiment, an equal or decreased level of sTWEAK compared to its reference value is indicative that the patient suffers from a high risk or intermediate risk prostate cancer or that there is a high likelihood that the patient develops high risk or intermediate risk prostate cancer; and an increased level of sTWEAK compared to its reference value is indicative that the patient suffers from low risk prostate cancer, or that there is a low likelihood that the patient develops a high risk or intermediate risk prostate cancer.

In a preferred embodiment, the reference value is obtained from sTWEAK in patients with low risk prostate cancer. In a more preferred embodiment, said reference value is obtained from plasma seminal samples. In an even more preferred embodiment, said sTWEAK levels in plasma seminal samples from low risk prostate cancer is between 300 and 1500 pg/mg protein, between 400 and 1400 pg/mg protein, between 500 and 1300 pg/mg protein, between 600 and 1200 pg/mg protein, between 700 and 1100 pg/mg protein, between 800 and 1000 pg/mg protein, between 900 and 950 pg/mg protein. In a still more preferred embodiment, sTWEAK level in plasma seminal samples from low risk prostate cancer patients is 931.8 ± 537.9 pg/mg protein.

The term "decreased level", in relation to the level of sTWEAK relates to any level of sTWEAK lower than the reference value. Thus, the sTWEAK level is considered to be decreased compared to its reference value when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more lower than its reference value.

The term "increased level", in relation to the level of sTWEAK relates to any level of sTWEAK higher than the reference value. Thus, the sTWEAK level is considered to be increased compared to its reference value when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than its reference value.

The term "equal level", in relation to the level of sTWEAK relates to any level of sTWEAK equal or substantially equal to the reference value. Thus, the sTWEAK level is considered to be equal compared to its reference value when is less than 5%, less than 4%, less than 3%, less than 2%, less than 1%, less than 0.5%, less than 0.1%, less than 0.05%, less than 0.01%, less than 0.001%, higher or lower than its reference value.

The term "high risk" or "high likelihood", as used herein, refers to a significant probability of suffering from or developing a particular condition. In a particular embodiment, a high likelihood is at least about 20%, including but not limited to about 25%, 30%, 35%, 40%, 45%, 50%, 55%, 60%, 65%, 70%, 75%, 80%, 85%, 90%, 95%, 100%, 150%, 200%, 300%, 400%, 500%, 600%, 700%, 800%, 900%, 1000%, and 1500%. In one particular embodiment, a high likelihood is at least 100%. In other embodiments, a high likelihood is at least 200%, at least 300%, at least 400%, at least 500%, at least 700%, at least 800%, at least 900% and at least 1000%. Other cut-offs or ranges as deemed suitable by the person skilled in the art to characterise the invention are however also contemplated, and those are also within the scope of the present invention.

The term "low risk" or "low likelihood", as used herein, refers to a not significant probability of suffering from or developing a particular condition. In a particular embodiment, a low likelihood is at least 5 %, at least 10%, at least 20%, at least 30%, at least 40%, at least 50%, at least 60%, at least 70%, at least 80%, at least 90%, at least 100% probabilities of not developing a particular condition.

The inventors have found that by including total PSA in blood and sTWEAK levels in semen in the first method of the invention, the patient classification is improved up to 77.1% with a sensitivity and specificity of 83.3% and 62.5%, respectively. Therefore, in a particular embodiment, the method further comprises:
(a) determining the level of total prostate specific antigen (PSA) protein in a blood sample from said patient and
(b) comparing said level with a reference value,
wherein
- an increased level of total PSA compared to the reference value is indicative that the patient suffers from high risk or intermediate risk prostate cancer, or that there is a high likelihood that the patient develops a high risk or intermediate risk prostate cancer, or, alternatively,
- a equal or decreased level of total PSA compared to the reference value is indicative that the patient suffers from low risk prostate cancer, or that there is a low likelihood that the patient develops a high risk or intermediate risk prostate cancer.

The term "prostate soluble antigen" or "PSA", as used herein, refers to a glycoprotein that in humans is encoded by the KLK3 gene. PSA hydrolyzes semenogelin-1 leading to the liquefaction of the seminal coagulum. PSA is present in small quantities in the serum of men with healthy prostates, but is often elevated in the presence of prostate cancer or other prostate disorders. In humans, PSA corresponds to the protein defined under accession number P07288 in UniProtKB/Swiss-Prot (Entry version 199 of 12 Sep 2018, Sequence version 2 of 1 July 1989).

The first method of the invention comprises determining the level of total PSA. The term "free PSA" refers to PSA that is unbound or not bound to another molecule. The term "bound PSA" refers to PSA that is bound to another molecule. The term "total PSA" refers to the sum of free PSA and bound PSA. The term "free:total PSA" or "F/T PSA" is the ratio of unbound PSA to total PSA.

The term "blood sample", as used herein, refers to biological material isolated from the blood of a subject. The blood sample can be whole blood, plasma or serum. In a preferred embodiment the blood sample is a serum sample.

The level of total PSA can be determined using any of the protein quantification techniques described before.

Total PSA reference level can be obtained as previously explained for sTWEAK reference level.

In a particular embodiment, the reference value is the level of total PSA in one or more subjects that are known to suffer from low risk prostate cancer. In this particular embodiment, an increased level of total PSA compared to its reference value is indicative that the patient suffers from a high risk or intermediate risk prostate cancer or that there is a high likelihood that the patient develops high risk or intermediate risk prostate cancer; and a equal or decreased level of total PSA compared to its reference value is indicative that the patient suffers from low risk prostate cancer, or that there is a low likelihood that the patient develops a high risk or intermediate risk prostate cancer.

In another particular embodiment, the reference value is the level of total PSA in one or more subjects who suffer from high or intermediate risk prostate cancer. In this particular embodiment, an equal or increased level of total PSA compared to the reference value is indicative that the patient suffers from a high risk or intermediate risk prostate cancer or that there is a high likelihood that the patient develops high risk or intermediate risk prostate cancer; and a decreased level of total PSA compared to the reference value is indicative that the patient suffers from low risk prostate cancer, or that there is a low likelihood that the patient develops a high risk or intermediate risk prostate cancer.

In a preferred embodiment, the reference value is obtained from total PSA in patients with low risk prostate cancer. In a more preferred embodiment, said reference value is obtained from blood serum. In an even more preferred embodiment, said total PSA level in blood serum samples from low risk prostate cancer is between 5.0 and 7.0 ng/mL, between 5.1 and 6.9 ng/mL, between 5.2 and 6.8 ng/mL, between 5.3 and 6.7 ng/mL, between 5.4 and 6.6 ng/mL, between 5.6 and 6.5 ng/mL, between 5.7 and 6.4 ng/mL, between 5.8 and 6.3 ng/mL, between 5.9 and 6.1 ng/mL. In a still more preferred embodiment, said total PSA level in blood serum samples from low risk prostate cancer is about 5.963 ± 0.333 ng/mL.

The terms "decreased level", "increased level" and "equal level" previously defined for sTWEAK fully apply to total PSA.

The inventors have also found that including sFN14 levels and sCD163 levels in seminal plasma and glucose levels in blood plasma together with sTWEAK levels in seminal plasma and total PSA levels in blood serum improve accuracy of the model, allowing correctly classifying 80% of patients with a sensitivity of 82.6% and a specificity of 72.7% (Figure 4C).

Therefore, in a particular embodiment, the method further comprises
(a) determining the level of at least one additional biomarker selected from the group consisting of: tumor necrosis factor receptor superfamily member 12A (FN14), soluble scavenger receptor cysteine-rich type 1 protein M130 (sCD163) and glucose, wherein the levels of FN14 and sCD163 are determined in a sample of semen and the level of glucose is determined in a blood sample and
(b) comparing said level with a reference value,
wherein
- a decreased level of FN14 and/or sCD163 compared to its reference value is indicative that the patient suffers from a high risk or intermediate risk prostate cancer, or that there is a high likelihood that the patient develops a high risk or intermediate risk prostate cancer, or, alternatively,
- an equal or increased level of FN14 and/or sCD163 compared to its reference value is indicative that the patient suffers from low risk prostate cancer, or that there is a low likelihood that the patient develops a high risk or intermediate risk prostate cancer.

The term "tumor necrosis factor receptor superfamily member 12A" or "FN14" or "TWEAK receptor", as used herein, refers to a protein that in humans is encoded by the TNFRSF12A gene. FN14 is the TWEAK receptor. In humans, FN14 corresponds to the protein defined under accession number Q9NP84 in UniProtKB/Swiss-Prot (Entry version 145 of 12 Sep 2018, Sequence version 1 of 1 October 2000). The FN14 receptor is the smallest TNFR superfamily member described so far. It is initially synthesized as a 129-amino-acid type I transmembrane protein that is then proteolytically processed by a signal peptidase into a 102-amino-acid cell-surface receptor. In contrast to the well-documented sTNF-R, the generation of soluble FN14 (sFN14) has not been formally reported. However, a soluble form of FN14 is described by Nusrat et al., PLoS One 2016, 11(5): e0155368. In a particular embodiment, FN14 is soluble FN14 or sFN14. The term "sFN14" or "soluble FN14" refers to the 102 amino protein originated by the proteolytic processing of FN14 as described by Nusrat et al.

The term "scavenger receptor cysteine-rich type 1 protein M130" or "CD163", as used herein, refers to a protein that in humans is encoded by the CD163 gene. In humans, CD163 corresponds to the protein defined under accession number Q86VB7 in UniProtKB/Swiss-Prot (Entry version 130 of 12 Sep 2018, Sequence version 2 of 30 November 2010). CD163 exists in two forms: a membrane associated form and a soluble form, which originates from the proteolytic shreding of the membrane form induced by lipopolysaccharide, phorbol ester and Fc region of immunoglobulin gamma. Therefore, the term "soluble CD163" or "sCD163", as used herein, refers to the processed from of CD163, which is not membrane associated.

The levels of FN14, particularly sFN14, and sCD163 can be determined using any of the protein quantification techniques described above.

According to the first method of the invention, the levels of FN14, particularly sFN14, and sCD163 are determined in a sample of semen, preferably in a sample of seminal plasma.

The term "glucose", as used herein, refers to a monosaccharide with the molecular formula C₆H₁₂O₆. Preferably, the glucose is D-glucose, which is the biologically active stereoisomer.

According to the first method of the invention, glucose levels are determined in a blood sample, preferably in a sample of blood plasma.

Glucose levels can be determined by any suitable and well-known technique for quantifying glucose levels in a blood sample, such methods based on the use of enzymes that, upon specifically reacting with the beta-D-glucose present in the blood sample, give rise to a compound that can be detected colorimetrically or spectrophotometrically. Illustrative non-limitative examples of said methods are glucose oxidase, hexokinase or glucose dehydrogenase methods. Average blood glucose concentrations can also be measured. This method measures the level of glycated hemoglobin, which is representative of the average blood glucose levels over the last, approximately, 120 days. In either case, the chemical system is commonly contained on a test strip which is inserted into a meter, and then has a blood sample applied. Test-strip shapes and their exact chemical composition vary between meter systems and cannot be interchanged. Formerly, some test strips were read (after timing and wiping away the blood sample) by visual comparison against a color chart printed on the vial label. Strips of this type are still used for urine glucose readings, but for blood glucose levels they are obsolete. Their error rates were, in any case, much higher. Errors when using test strips were often caused by the age of the strip or exposure to high temperatures or humidity.

In a particular embodiment, the first method of the invention comprises determining the level of sTWEAK in a sample of semen and the level of just one additional biomarker selected from the group consisting of FN14, particularly sFN14, in a sample of semen, sCD163 in a sample of semen and glucose in a blood sample. In a more particular embodiment, the additional biomarker is FN14, particularly sFN14, in a sample of semen. In another more particular embodiment, the additional biomarker is sCD163 in a sample of semen. In another more particular embodiment, the additional biomarker is glucose in a blood sample.

In a particular embodiment, the first method of the invention comprises determining the level of sTWEAK in a sample of semen and the level of two additional biomarkers selected from the group consisting of FN14, particularly sFN14, in a sample of semen, sCD163 in a sample of semen and glucose in a blood sample. In a more particular embodiment, the two additional biomarkers are FN14, particularly sFN14, and sCD163 in a sample of semen. In another more particular embodiment, the additional biomarker is sCD163 in a sample of semen and glucose in a blood sample. In another more particular embodiment, the two additional biomarkers are FN14, particularly sFN14, in a sample of semen and glucose in a blood sample.

In a particular embodiment, the first method of the invention comprises determining the level of sTWEAK in a sample of semen and the level of the three additional biomarkers FN14, particularly sFN14, in a sample of semen, sCD163 in a sample of semen and glucose in a blood sample.

In a particular embodiment, the first method of the invention comprises determining the level of sTWEAK in a sample of semen, the level of total PSA in a blood sample and the level of just one additional biomarker selected from the group consisting of FN14, particularly sFN14, in a sample of semen, sCD163 in a sample of semen and glucose in a blood sample. In a more particular embodiment, the additional biomarker is FN14, particularly sFN14, in a sample of semen. In another more particular embodiment, the additional biomarker is sCD163 in a sample of semen. In another more particular embodiment, the additional biomarker is glucose in a blood sample.

In a particular embodiment, the first method of the invention comprises determining the level of sTWEAK in a sample of semen, the level of total PSA in a blood sample and the level of two additional biomarkers selected from the group consisting of FN14, particularly sFN14, in a sample of semen, sCD163 in a sample of semen and glucose in a blood sample. In a more particular embodiment, the two additional biomarkers are FN14, particularly sFN14, and sCD163 in a sample of semen. In another more particular embodiment, the additional biomarker is sCD163 in a sample of semen and glucose in a blood sample. In another more particular embodiment, the two additional biomarkers are FN14, particularly sFN14, in a sample of semen and glucose in a blood sample.

In a particular embodiment, the first method of the invention comprises determining the level of sTWEAK in a sample of semen, the level of total PSA in a blood sample and the level of the three additional biomarkers FN14, particularly sFN14, in a sample of semen, sCD163 in a sample of semen and glucose in a blood sample.

In a particular embodiment, the reference value for FN14, particularly sFN14, is the FN14, particularly sFN14, level in one or more subjects known to suffer from low risk prostate cancer. In this particular embodiment, a decreased level of FN14, particularly sFN14, compared to its reference value is indicative that the patient suffers from a high risk or intermediate risk prostate cancer or that there is a high likelihood that the patient develops high risk or intermediate risk prostate cancer; and an equal or increased level of FN14, particularly sFN14, compared to its reference value is indicative that the patient suffers from low risk prostate cancer, or that there is a low likelihood that the patient develops a high risk or intermediate risk prostate cancer.

In another particular embodiment, the reference value for FN14, particularly sFN14, is the FN14, particularly sFN14, level in one or more subjects who suffer from high or intermediate risk prostate cancer. In this particular embodiment, an equal or decreased level of FN14, particularly sFN14, compared to its reference value is indicative that the patient suffers from a high risk or intermediate risk prostate cancer or that there is a high likelihood that the patient develops high risk or intermediate risk prostate cancer; and an increased level of FN14, particularly sFN14, compared to its reference value is indicative that the patient suffers from low risk prostate cancer, or that there is a low likelihood that the patient develops a high risk or intermediate risk prostate cancer.

In a preferred embodiment, the reference value for FN14, particularly sFN14, is obtained from patients with low risk prostate cancer. In a more preferred embodiment, said reference value is obtained from plasma seminal samples. In an even more preferred embodiment, said FN14, particularly sFN14, level in plasma seminal samples from low risk prostate cancer is between 200 and 1200 pg/mg, between 300 and 1100 pg/mg, between 400 and 1000 pg/mg, between 500 and 900 pg/mg, between 600 and 800 pg/mg, between 650 and 700 pg/mg. In a still more preferred embodiment, said FN14, particularly sFN14, level in plasma seminal samples from low risk prostate cancer is about 694.8±451.1 pg/mg protein.

In a particular embodiment, the reference value for sCD163 is the sCD163 level in one or more subjects known to suffer from low risk prostate cancer. In this particular embodiment, a decreased level of sCD163 compared to its reference value is indicative that the patient suffers from a high risk or intermediate risk prostate cancer or that there is a high likelihood that the patient develops high risk or intermediate risk prostate cancer; and an equal or increased level of sCD163 compared to its reference value is indicative that the patient suffers from low risk prostate cancer, or that there is a low likelihood that the patient develops a high risk or intermediate risk prostate cancer.

In another particular embodiment, the reference value for sCD163 is the sCD163 level in one or more subjects who suffer from high or intermediate risk prostate cancer. In this particular embodiment, an equal or decreased level of sCD163 compared to its reference value is indicative that the patient suffers from a high risk or intermediate risk prostate cancer or that there is a high likelihood that the patient develops high risk or intermediate risk prostate cancer; and an increased level of sCD163 compared to its reference value is indicative that the patient suffers from low risk prostate cancer, or that there is a low likelihood that the patient develops a high risk or intermediate risk prostate cancer.

In a preferred embodiment, the reference value for sCD163 is obtained from patients with low risk prostate cancer. In a more preferred embodiment, said reference value is obtained from plasma seminal samples. In an even more preferred embodiment, said sCD163 level in plasma seminal samples from low risk prostate cancer is between 800 and 4000 pg/protein, between 900 and 3000 pg/protein, between 1000 and 2800 pg/protein, between 1100 and 2500 pg/protein, between 1200 and 2200 pg/protein, between 1300 and 2000 pg/protein, between 1400 and 1800 pg/protein, between 1500 and 1600 pg/protein.

In a particular embodiment, the reference value for glucose is the glucose level in one or more subjects known to suffer from low risk prostate cancer. In this particular embodiment, a decreased level of glucose compared to its reference value is indicative that the patient suffers from a high risk or intermediate risk prostate cancer or that there is a high likelihood that the patient develops high risk or intermediate risk prostate cancer; and an equal or increased level of glucose compared to its reference value is indicative that the patient suffers from low risk prostate cancer, or that there is a low likelihood that the patient develops a high risk or intermediate risk prostate cancer. In another particular embodiment, an increased level of glucose compared to its reference value is indicative that the patient suffers from a high risk or intermediate risk prostate cancer or that there is a high likelihood that the patient develops high risk o intermediate risk prostate cancer, and an equal or decreased level of glucose compared to its reference value is indicative that the patient suffers from low risk prostate cancer, or that there is a low likelihood that the patient develops a high risk or intermediate risk prostate cancer.

In another particular embodiment, the reference value for glucose is the glucose level in one or more subjects who suffer from high or intermediate risk prostate cancer. In this particular embodiment, an equal or increased level of glucose compared to its reference value is indicative that the patient suffers from a high risk or intermediate risk prostate cancer or that there is a high likelihood that the patient develops high risk or intermediate risk prostate cancer; and a decreased level of glucose compared to its reference value is indicative that the patient suffers from low risk prostate cancer, or that there is a low likelihood that the patient develops a high risk or intermediate risk prostate cancer. In a particular embodiment, an equal or decreased level of glucose compared to its reference value is indicative that the patient suffers from a high risk or intermediate risk prostate cancer or that there is a high likelihood that the patient develops high risk or intermediate risk prostate cancer; and an increased level of glucose compared to its reference value is indicative that the patient suffers from low risk prostate cancer, or that there is a low likelihood that the patient develops a high risk or intermediate risk prostate cancer.

In a preferred embodiment, the reference for glucose is obtained from patients with low risk prostate cancer. In a more preferred embodiment, said reference value is obtained from serum blood glucose levels.

The terms "decreased level", "increased level" and "equal level" previously defined for sTWEAK fully apply to sFN14, sCD163 and glucose.

### Methods of personalized therapy

In another aspect, the invention relates to a method for selecting a therapy for a patient suffering from prostate cancer, hereinafter method for selecting a therapy of the invention, comprising:
(a) determining the level of sTWEAK in a sample of semen from the patient and
(b) comparing said level with a reference value
wherein
- if a decreased level of sTWEAK compared to the reference value is detected, a therapy selected from the group consisting of prostate surgery, radiotherapy and a combination thereof is selected or, alternatively,
- if an equal or increased level of sTWEAK compared to the reference value is detected, an alternative therapy is selected.

The terms "patient", "prostate cancer", "sTWEAK", "sample of semen", "reference value", "decreased, increased and equal level" have been previously defined.
The term "therapy" or "treatment", as used herein, refers to the attempted remediation of a health problem, usually following a diagnosis, or to prevention of the appearance of a health problem. As such, it is not necessarily a cure, i.e., a complete reversion of a disease. Said therapy may or may not be known to have a positive effect on a particular disease. This term includes both therapeutic treatment and prophylactic or preventive measures, in which the object is to prevent or stop (reduce) an undesired physiological change or disorder. For the purpose of this invention, beneficial or desired clinical results include, without limitation, relieving symptoms, reducing the spread of the disease, stabilizing pathological state (specifically not worsening), slowing down or stopping the progression of the disease, improving or mitigating the pathological state and remission (both partial and complete), both detectable and undetectable. It can also involve prolonging survival, disease free survival and symptom free survival, in comparison with the expected survival if treatment is not received. Those subjects needing treatment include those subjects already suffering the condition or disorder, as well as those with the tendency to suffer the condition or disorder or those in which the condition or disorder must be prevented.

According to the method for selecting a therapy of the invention, when the prostate cancer patient has decreased levels of sTWEAK in a sample of semen compared to a reference value, a therapy is selected from the group consisting of prostate surgery, radiotherapy and a combination thereof.

The term "prostate surgery" or "prostatectomy", as used herein, refers to the surgical removal of the prostate gland and some surrounding tissue. The most common type of prostate surgery is radical prostatectomy, wherein the entire prostate gland is removed, optionally together with the seminal vesicles and the nearby lymph nodes. Radical prostatectomy can be an open surgery or minimally invasive surgery (also called keyhole or laparoscopy surgery), which can be performed by hand or be robot-assisted.

The term "chemotherapy" refers to the use of drugs to destroy cancer cells. The drugs are generally administered through oral or intravenous route. Sometimes, chemotherapy is used together with radiation treatment. Suitable chemotherapy agents include but are not limited to alkylating agents [e.g., Cisplatin, Carboplatin, Oxaliplatin, BBR3464, Chlorambucil, Chlormethine, Cyclophosphamides, Ifosfamide, Melphalan, Carmustine, Fotemustine, Lomustine, Streptozocin, Busulfan, Dacarbazine, Mechlorethamine, Procarbazine, Temozolomide, ThioTPA, Uramustine, etc.]; antimetabolites [e.g., purine (azathioprine, mercaptopurine), pyrimidine (Capecitabine, Cytarabine, Fluorouracil, Gemcitabine), folic acid (Methotrexate, Pemetrexed, Raltitrexed), etc.]; vinca alkaloids [e.g., Vincristine, Vinblastine, Vinorelbine, Vindesine, etc.]; a taxane [e.g., paclitaxel, docetaxel, BMS-247550, etc.]; an anthracycline [e.g., Daunorubicin, Doxorubicin, Epirubicin, Idarubicin, Mitoxantrone, Valrubicin, Bleomycin, Hydroxyurea, Mitomycin, etc.]; a topoisomerase inhibitor [e.g., Topotecan, Irinotecan Etoposide, Teniposide, etc.]; a monoclonal antibody [e.g., Alemtuzumab, Bevacizumab, Cetuximab, Gemtuzumab, Panitumumab, Rituximab, Trastuzumab, etc.]); a photosensitizer [e.g., Aminolevulinic acid, Methyl aminolevulinate, Porfimer sodium, Verteporfin, etc.]; a tyrosine kinase inhibitor [e.g., imatinib]; an epidermal growth factor receptor inhibitor [e.g., erlotinib, gefitinib, etc.]; an FPTase inhibitor [e.g., FTIs (R1 15777, SCH66336, L-778, 123), etc.]; a KDR inhibitor [e.g., SU6668, PTK787, etc.]; a proteosome inhibitor [e.g., PS341, etc.]; a TS/DNA synthesis inhibitor [e.g., ZD9331, Raltitrexed (ZD 1694, Tomudex), ZD9331, 5-FU, etc.]; an S-adenosyl-methionine decarboxylase inhibitor [e.g., SAM468A, etc.]; a DNA methylating agent [e.g., TMZ, etc.]; a DNA binding agent [e.g., PZA, etc.]; an agent which binds and inactivates O-6-alkylguanine AGT [e.g., BG]; a c-ra/-1 antisense oligo-deoxynucleotide [e.g., ISIS-5132 (CGP- 69846A)]; tumor immunotherapy; a steroidal and/or nonsteroidal antiinflammatory agent [e.g., corticosteroids, COX-2 inhibitors]; or other agents such as Alitretinoin, Altretamine, Amsacrine, Anagrelide, Arsenic trioxide, Asparaginase, Bexarotene, Bortezomib, Celecoxib, Dasatinib, Denileukin Diftitox, Estramustine, Hydroxycarbamide, Imatinib, Pentostatin, Masoprocol, Mitotane, Pegaspargase, and Tretinoin. Two pivotal studies have proven that the survival of patients with metastatic prostate cancer resistant to castration may improve after intravenous docetaxel administration every 3 weeks (Petrylak et al., N. Engl. J. Med. 2004, 351(15): 1513-1520; Tannock et al., N. Engl. J. Med. 2004, 351(15): 1502-1512) Therefore, in a particular embodiment, the chemotherapy agent is docetaxel.

When the prostate cancer patient has equal or increased levels of sTWEAK in a sample of semen compared to the reference value, the therapy selected is an alternative therapy, that is, a therapy other than prostate surgery, chemotherapy, radiotherapy or a combination thereof. Illustrative non-limitative examples of alternative therapies are watchfaul waiting and active surveillance.

The term "watchful waiting", as used herein, refers to a way of monitoring a prostate cancer that is not causing any symptoms or problems. The aim is to avoid treatments that may have underside side effects in a cancer that often slow grow and no causing problems and symptoms.

The term "active surveillance", as used herein, refers to a treatment plan that involves closely watching a patient's condition but not giving any treatment unless there are changes in test results that show the condition is getting worse. Active surveillance may be used to avoid or delay the need for treatments such as radiation therapy or surgery, which can cause side effects or other problems. During active surveillance, certain exams and tests are done on a regular schedule. It is a type of expectant management.

Watchful waiting and active surveillance have both the aim of avoiding unnecessary treatments, but differ in the following aspects:
- Watchful waiting usually involves fewer tests than active surveillance and the tests usually are not invasive.
- Watchful waiting is generally suitable for men with other health problems who may be less able to cope with treatments such as surgery or radiotherapy, or whose cancer may never cause problems during their lifetime. On the contrary, active surveillance is suitable for most men with cancer that is contained in the prostate (localised cancer), and who could cope with treatments such as surgery or radiotherapy if they needed it.
- In case a treatment is needed at any point, it is usually aimed to control the cancer rather than cure it in case of watchful waiting, and to cure it in case of active surveillance.

In another aspect, the invention is directed to a method for selecting a patient suffering from prostate cancer for a treatment selected from the group consisting of prostate surgery, radiotherapy and a combination thereof, hereinafter method for selecting a patient of the invention, comprising:
(a) determining the level of sTWEAK in a sample of semen from the patient and
(b) comparing said level with a reference value
wherein the patient is selected for said treatment if a decreased level of sTWEAK compared to the reference value is detected.

The terms "patient", "prostate cancer", "sTWEAK", "sample of semen", "reference value", "decreased, increased and equal level", "prostate surgery" and "radiotherapy" have been previously defined.

In a particular embodiment, the method for selecting a therapy and the method for selecting a patient further comprise
(a) determining the level of total PSA in a blood sample from said patient and
(b) comparing said level with its reference value,
wherein
- if an increased level of total PSA compared to its reference value is detected, a therapy selected from the group consisting of prostate surgery, radiotherapy and a combination thereof is selected, or the patient is selected for said treatment, or, alternatively,
- if an equal or decreased level of total PSA compared to its reference value is detected, an alternative therapy is selected.

The term "total PSA" has been previously defined.

The term "blood sample" has been previously defined. In a preferred embodiment, the blood sample is a plasma sample.

The term "reference value" for total PSA has been previously defined.

In a particular embodiment, when the reference value is the level of total PSA in one or more subjects that are known to suffer from low risk prostate cancer, if an increased level of total PSA compared to its reference value is detected, a therapy selected from the group consisting of prostate surgery, radiotherapy and a combination thereof is selected, or the patient is selected for said treatment; and if an equal or decreased level of total PSA compared to its reference value is detected, an alternative therapy is selected.

In another particular embodiment, when the reference value is the level of total PSA in one or more subjects who suffer from high or intermediate risk prostate cancer, if an equal or increased level of total PSA compared to the reference value is detected, a therapy selected from the group consisting of prostate surgery, radiotherapy and a combination thereof is selected, or the patient is selected for said treatment; and if a decreased level of total PSA compared to the reference value is detected, an alternative therapy is selected.

In a preferred embodiment, the reference value is obtained from total PSA in patients with low risk prostate cancer. In a more preferred embodiment, said reference value is obtained from blood serum. In an even more preferred embodiment, said total PSA level in blood serum samples from low risk prostate cancer is 5.963 ± 0.333 ng/mL.

In a particular embodiment, the method for selecting a therapy and the method for selecting a patient further comprises
(a) determining the level of at least one additional biomarker selected from the group consisting of FN14, particularly sFN14, sCD163 and glucose, wherein the levels of FN14, particularly sFN14, and sCD163 are determined in a sample of semen and the level of glucose is determined in a blood sample and
(b) comparing said level with a reference value,
wherein
- if a decreased level of FN14, particularly sFN14, and/or sCD163 compared to its reference value is detected, a therapy selected from the group consisting of prostate surgery, radiotherapy and a combination thereof is selected, or the patient is selected for said treatment, or, alternatively,
- if an equal or increased level of FN14, particularly sFN14, and/or sCD163 compared to its reference value is detected, an alternative therapy is selected.

The terms "FN14", "sFN14", "sCD163", and "glucose" have been previously defined.

According to the method for selecting a therapy and for selecting a patient of the invention, the levels of FN14, particularly sFN14, and sCD163 are determined in a sample of semen, preferably in a sample of seminal plasma, and the levels of glucose are determined in a blood sample, preferably in a sample of blood plasma.

In a particular embodiment, the methods for selecting a therapy and for selecting a patient of the invention comprise determining the level of sTWEAK in a sample of semen and the level of just one additional biomarker selected from the group consisting of FN14, particularly sFN14, in a sample of semen, sCD163 in a sample of semen and glucose in a blood sample. In a more particular embodiment, the additional biomarker is FN14, particularly sFN14, in a sample of semen. In another more particular embodiment, the additional biomarker is sCD163 in a sample of semen. In another more particular embodiment, the additional biomarker is glucose in a blood sample.

In a particular embodiment, the methods for selecting a therapy and for selecting a patient of the invention comprise determining the level of sTWEAK in a sample of semen and the level of two additional biomarkers selected from the group consisting of FN14, particularly sFN14, in a sample of semen, sCD163 in a sample of semen and glucose in a blood sample. In a more particular embodiment, the two additional biomarkers are FN14, particularly sFN14, and sCD163 in a sample of semen. In another more particular embodiment, the additional biomarker is sCD163 in a sample of semen and glucose in a blood sample. In another more particular embodiment, the two additional biomarkers are FN14, particularly sFN14, in a sample of semen and glucose in a blood sample.

In a particular embodiment, the methods for selecting a therapy and for selecting a patient of the invention comprise determining the level of sTWEAK in a sample of semen and the level of the three additional biomarkers FN14, particularly sFN14, in a sample of semen, sCD163 in a sample of semen and glucose in a blood sample.

In a particular embodiment, the methods for selecting a therapy and for selecting a patient of the invention comprise determining the level of sTWEAK in a sample of semen, the level of total PSA in a blood sample and the level of just one additional biomarker selected from the group consisting of FN14, particularly sFN14, in a sample of semen, sCD163 in a sample of semen and glucose in a blood sample. In a more particular embodiment, the additional biomarker is FN14, particularly sFN14, in a sample of semen. In another more particular embodiment, the additional biomarker is sCD163 in a sample of semen. In another more particular embodiment, the additional biomarker is glucose in a blood sample.

In a particular embodiment, the methods for selecting a therapy and for selecting a patient of the invention comprise determining the level of sTWEAK in a sample of semen, the level of total PSA in a blood sample and the level of two additional biomarkers selected from the group consisting of FN14, particularly sFN14, in a sample of semen, sCD163 in a sample of semen and glucose in a blood sample. In a more particular embodiment, the two additional biomarkers are FN14, particularly sFN14, and sCD163 in a sample of semen. In another more particular embodiment, the additional biomarker is sCD163 in a sample of semen and glucose in a blood sample. In another more particular embodiment, the two additional biomarkers are FN14, particularly sFN14, in a sample of semen and glucose in a blood sample.

In a particular embodiment, the methods for selecting a therapy and for selecting a patient of the invention comprise determining the level of sTWEAK in a sample of semen, the level of total PSA in a blood sample and the level of the three additional biomarkers FN14, particularly sFN14, in a sample of semen, sCD163 in a sample of semen and glucose in a blood sample.

In a particular embodiment, when the reference value for FN14, particularly sFN14, is the FN14, particularly sFN14, level in one or more subjects known to suffer from low risk prostate cancer, if a decreased level of FN14, particularly sFN14, compared to its reference value is detected, a therapy selected from the group consisting of prostate surgery, radiotherapy and a combination thereof is selected, or the patient is selected for said therapy; and if an equal or increased level of FN14, particularly sFN14, compared to its reference value is detected, an alternative therapy is selected.

In another particular embodiment, when the reference value for FN14, particularly sFN14, is the FN14, particularly sFN14, level in one or more subjects who suffer from high or intermediate risk prostate cancer, if an equal or decreased level of FN14, particularly sFN14, compared to its reference value is detected, a therapy selected from the group consisting of prostate surgery, radiotherapy and a combination thereof is selected, or the patient is selected for said therapy; and if an increased level of FN14, particularly sFN14, compared to its reference value is detected, an alternative therapy is selected.

In a preferred embodiment, the reference value for FN14, particularly sFN14, is obtained from patients with low risk prostate cancer. In a more preferred embodiment, said reference value is obtained from plasma seminal samples. In an even more preferred embodiment, said FN14, particularly sFN14, level in plasma seminal samples from low risk prostate cancer is 694.8±451.1 pg/mg protein.

In a particular embodiment, when the reference value for sCD163 is the sCD163 level in one or more subjects known to suffer from low risk prostate cancer, if a decreased level of sCD163 compared to its reference value is detected, a therapy selected from the group consisting of prostate surgery, radiotherapy and a combination thereof is selected, or the patient is selected for said therapy; and if an equal or increased level of sCD163 compared to its reference value is detected, an alternative therapy is selected.

In another particular embodiment, when the reference value for sCD163 is the sCD163 level in one or more subjects who suffer from high or intermediate risk prostate cancer, if an equal or decreased level of sCD163 compared to its reference value is detected, a therapy selected from the group consisting of prostate surgery, radiotherapy and a combination thereof is selected, or the patient is selected for said therapy, and if an increased level of sCD163 compared to its reference value is detected, an alternative therapy is selected.

In a preferred embodiment, the reference value for sCD163 is obtained from patients with low risk prostate cancer. In a more preferred embodiment, said reference value is obtained from plasma seminal samples. In an even more preferred embodiment, said sCD163 level in plasma seminal samples from low risk prostate cancer is 1521±2410 pg/mg protein.

In a particular embodiment, when the reference value for glucose is the glucose level in one or more subjects known to suffer from low risk prostate cancer, if a decreased level of glucose compared to its reference value is detected, a therapy selected from the group consisting of prostate surgery, radiotherapy and a combination thereof is selected, or the patient is selected for said therapy; and if an equal or increased level of glucose compared to its reference value is detected, an alternative therapy is selected. In another particular embodiment, when the reference value for glucose is the glucose level in one or more subjects known to suffer from low risk prostate cancer, if an increased level of glucose compared to its reference value is detected, a therapy selected from the group consisting of prostate surgery, radiotherapy and a combination thereof is selected, or the patient is selected for said therapy; and if an equal or decreased level of glucose compared to its reference value detected, an alternative therapy is selected.

In another particular embodiment, when the reference value for glucose is the glucose level in one or more subjects who suffer from high or intermediate risk prostate cancer, if an equal or increased level of glucose compared to its reference value is detected, a therapy selected from the group consisting of prostate surgery, radiotherapy and a combination thereof is selected, or the patient is selected for said therapy; and if a decreased level of glucose compared to its reference value is detected, an alternative therapy is selected. In another particular embodiment, when the reference value for glucose is the glucose level in one or more subjects who suffer from high or intermediate risk prostate cancer, if an equal or decreased level of glucose compared to its reference value is detected, a therapy selected from the group consisting of prostate surgery, radiotherapy and a combination thereof is selected, or the patient is selected for said therapy; and if an increased level of glucose compared to its reference value is detected, an alternative therapy is selected.

In a preferred embodiment, the reference for glucose is obtained from patients with low risk prostate cancer. In a more preferred embodiment, said reference value is obtained from serum blood glucose levels.

### Kits of the invention and uses thereof

In another aspect, the invention relates to a kit comprising a reagent specific for determining the level of sTWEAK protein and at least an additional reagent specific for determining the level of a biomarker selected from the group consisting of total PSA, FN14, particularly sFN14, sCD163 and glucose.

The terms "sTWEAK", "total PSA", "FN14", "sFN14", "sCD163" and "glucose" have been previously defined.

The term "kit", as used herein, refers to a product containing the different reagents required for carrying out the methods of the invention packaged so as to allow their transport and storage. Additionally, the kits of the invention can contain instructions for the simultaneous, sequential or separate use of the different components in the kit. Said instructions can be found in the form of a printed material or in the form of an electronic support capable of storing instructions such that they can be read by a subject, such as electronic storage media (magnetic discs, tapes and the like), optical media (CD-ROM, DVD) and the like.

The term "specific reagent", as used herein, refers to any reagent that can be used for the quantification of the proteins sTWEAK, total PSA, FN14, particularly sFN14, or sCD163 or for quantification of glucose.

In a particular embodiment, the reagents specific for determining the levels of sTWEAK, total PSA, FN14, particularly sFN14, or sCD163 are compounds that specifically bind to these proteins. In a more particular embodiment, said reagents are antibodies, aptamers or fragments thereof.

In a preferred embodiment, the reagent is an antibody or fragment thereof.

The antibodies of the kit of the invention can be used according to techniques known in the art for determining the protein levels, such as, for example, flow cytometry, Western blot, ELISA, RIA, competitive EIA, DAS-ELISA, techniques based on the use of biochips, protein microarrays, or assays of colloidal precipitation in reactive strips.

In a particular embodiment, the reagent specific for determining the level of glucose is an enzyme used for specific quantification of blood glucose, such as glucose oxidase, hexokinase or glucose dehydrogenase.

In a particular embodiment, the kit of the invention comprises a reagent specific for determining the level of sTWEAK and just one additional reagent specific for determining the level of a biomarker selected from the group consisting of total PSA, FN14, particularly sFN14, sCD163 and glucose. In a more particular embodiment, the just one additional reagent is a reagent specific for determining the level of total PSA. In another more particular embodiment, the just one additional reagent is a reagent specific for determining the level of FN14, particularly sFN14. In another more particular embodiment, the just one additional reagent is a reagent specific for determining the level of sCD163. In another more particular embodiment, the just one additional reagent is a reagent specific for determining the level of glucose.

In a particular embodiment, the kit of the invention comprises a reagent specific for determining the level of sTWEAK and two additional reagents specific for determining the level of a biomarker selected from the group consisting of total PSA, FN14, particularly sFN14, sCD163 and glucose. In another more particular embodiment, the two additional reagents are reagents specific for determining the level of total PSA and FN14, particularly sFN14. In another more particular embodiment, the two additional reagents are reagents specific for determining the level of total PSA and sCD163. In another more particular embodiment, the two additional reagents are reagents specific for determining the level of total PSA and glucose. In another more particular embodiment, the two additional reagents are reagents specific for determining the level of FN14, particularly sFN14, and sCD163. In another more particular embodiment, the two additional reagents are reagents specific for determining the level of FN14, particularly sFN14, and glucose. In another more particular embodiment, the two additional reagents are reagents specific for determining the level of sCD163 and glucose.

In a particular embodiment, the kit of the invention comprises a reagent specific for determining the level of sTWEAK and three additional reagents specific for determining the level of a biomarker selected from the group consisting of total PSA, FN14, particularly sFN14, sCD163 and glucose. In another more particular embodiment, the three additional reagents are reagents specific for determining the level of total PSA, FN14, particularly sFN14, and sCD163. In another more particular embodiment, the three additional reagents are reagents specific for determining the level of total PSA, FN14, particularly sFN14, and glucose. In another more particular embodiment, the three additional reagents are reagents specific for determining the level of FN14, particularly sFN14, sCD163 and glucose.

In a particular embodiment, the kit of the invention comprises a reagent specific for determining the level of sTWEAK and the four additional reagents specific for determining the level of the biomarkers total PSA, FN14, particularly sFN14, sCD163 and glucose.

In a particular embodiment, the kit of the invention comprises a reagent specific for determining the level of sTWEAK, a reagent specific for determining the level of total PSA and one or more additional reagents specific for determining the level of a biomarker selected from the group consisting of FN14, particularly sFN14, sCD163 and glucose, including the following combinations: FN14, particularly sFN14; sCD163; glucose; FN14, particularly sFN14, and sCD163; FN14, particularly sFN14, and glucose; sCD163 and glucose; FN14, particularly sFN14, sCD163 and glucose.

In a particular embodiment of the kit of the invention, at least 5%, 10%, 20%, 30%, 40%, 50%, 60%, 70%, 80%, 90%, 95%, 99% or the 100% of the reagents specific for determining biomarkers comprised in the kit are reagents specific for determining the level of sTWEAK and any combination of the biomarkers total PSA, FN14, particularly sFN14, sCD163 and glucose, including the following combinations: total PSA; FN14, particularly sFN14; sCD163; glucose; FN14, particularly sFN14, and sCD163; FN14, particularly sFN14, and glucose; sCD163 and glucose; total PSA and FN14, particularly sFN14; total PSA and sCD163; total PSA and glucose; total PSA, FN14, particularly sFN14, and sCD163; total PSA, FN14, particularly sFN14, and glucose; total PSA, scD163 and glucose; FN14, particularly sFN14, sCD163 and glucose; total PSA, FN14, particularly sFN14, sCD163 and glucose.

In another aspect, the invention relates to the use of the kit of the invention for:
(a) the differential diagnosis of high risk or intermediate risk prostate cancer from low risk prostate cancer; or
(b) for determining the likelihood that a patient with prostate cancer develops a high risk or intermediate risk prostate cancer; or
(c) for selecting a therapy for a patient suffering from prostate cancer; or
(d) for selecting a patient suffering from prostate cancer for a treatment selected from the group consisting of prostate surgery, radiotherapy and a combination thereof.

All the terms included in the use of the kit of the invention have been previously defined.

### Additional aspects of the invention

1. An *in vitro* method for the differential diagnosis of high risk or intermediate risk prostate cancer from low risk prostate cancer, or for determining the likelihood that a patient with prostate cancer develops a high risk or intermediate risk prostate cancer, comprising:
   (a) determining the level of soluble tumor necrosis factor ligand superfamily member 12 (sTWEAK) protein in a sample of semen from the patient and
   (b) comparing said level with a reference value
   wherein
   - a decreased level of sTWEAK compared to the reference value is indicative that the patient suffers from a high risk or intermediate risk prostate cancer, or that there is a high likelihood that the patient develops a high risk or intermediate risk prostate cancer, or, alternatively,
   - an equal or increased level of sTWEAK compared to the reference value is indicative that the patient suffers from low risk prostate cancer, or that there is a low likelihood that the patient develops a high risk or intermediate risk prostate cancer.
2. The method according to aspect 1, further comprising
   (a) determining the level of total prostate specific antigen (PSA) protein in a blood sample from said patient and
   (b) comparing said level with a reference value,
   wherein
   - an increased level of total PSA compared to the reference value is indicative that the patient suffers from high risk or intermediate risk prostate cancer, or that there is a high likelihood that the patient develops a high risk or intermediate risk prostate cancer, or, alternatively,
   - an equal or decreased level of total PSA compared to the reference value is indicative that the patient suffers from low risk prostate cancer, or that there is a low likelihood that the patient develops a high risk or intermediate risk prostate cancer.
3. The method according to aspect 2, wherein the blood sample is a sample of serum.
4. The method according to any one of aspects 1 to 3, further comprising:
   (a) determining the level of at least one additional biomarker selected from the group consisting of: tumor necrosis factor receptor superfamily member 12A (FN14), particularly sFN14, soluble scavenger receptor cysteine-rich type 1 protein M130 (sCD163) and glucose, wherein the levels of FN14, particularly sFN14, and sCD163 are determined in a sample of semen and the level of glucose is determined in a blood sample and
   (b) comparing said level with a reference value,
   wherein
   - a decreased level of FN14, particularly sFN14, and/or sCD163 compared to its reference value is indicative that the patient suffers from a high risk or intermediate risk prostate cancer, or that there is a high likelihood that the patient develops a high risk or intermediate risk prostate cancer, or, alternatively,
   - an equal or increased level of FN14, particularly sFN14, and/or sCD163 compared to its reference value is indicative that the patient suffers from low risk prostate cancer, or that there is a low likelihood that the patient develops a high risk or intermediate risk prostate cancer.
5. The method according to aspect 4, wherein the level of glucose is determined in a sample of plasma.
6. The method according to any one of aspects 1 to 5, wherein the sample of semen is a sample of seminal plasma.
7. The method according to aspect 6, wherein the levels of sTWEAK, total PSA, FN14, particularly sFN14, sCD163 and glucose are determined.
8. The method according to any one of aspects 4 to 7, wherein the levels of FN14 are the levels of soluble FN14 (sFN14).
9. A method for selecting a therapy for a patient suffering from prostate cancer, comprising:
   (a) determining the level of sTWEAK in a sample of semen from the patient and
   (b) comparing said level with a reference value
   wherein
   - if a decreased level of sTWEAK compared to the reference value is detected, a therapy selected from the group consisting of prostate surgery, radiotherapy and a combination thereof is selected or, alternatively,
   - if an equal or increased level of sTWEAK compared to the reference value is detected, an alternative therapy is selected.
10. A method for selecting a patient suffering from prostate cancer for a treatment selected from the group consisting of prostate surgery, radiotherapy and a combination thereof, comprising:
   (a) determining the level of sTWEAK in a sample of semen from the patient and
   (b) comparing said level with a reference value
   wherein the patient is selected for said treatment if a decreased level of sTWEAK compared to the reference value is detected.
11. The method according to any one of aspects 9 or 10, further comprising
   (a) determining the level of total PSA in a blood sample from said patient and
   (b) comparing said level with its reference value,
   wherein
   - if an increased level of total PSA compared to its reference value is detected, a therapy selected from the group consisting of prostate surgery, radiotherapy and a combination thereof is selected, or the patient is selected for said treatment, or, alternatively,
   - if an equal or decreased level of total PSA compared to its reference value is detected, an alternative therapy is selected.
12. The method according to aspect 11, wherein the blood sample is a sample of serum.
13. The method according to any one of aspects 9 to 12, further comprising:
   (a) determining the level of at least one additional biomarker selected from the group consisting of FN14, sCD163 and glucose, wherein the levels of FN14 and sCD163 are determined in a sample of semen and the level of glucose is determined in a blood sample and
   (b) comparing said level with a reference value,
   wherein
   - if a decreased level of FN14 and/or sCD163 compared to its reference value is detected, a therapy selected from the group consisting of prostate surgery, radiotherapy and a combination thereof is selected, or the patient is selected for said treatment, or, alternatively,
   - if an equal or increased level of FN14 and/or sCD163 compared to its reference value is detected, an alternative therapy is selected.
14. The method according to aspect 13, wherein the level of glucose is determined in a sample of plasma.
15. The method according to any one of aspects 9 to 14, wherein the sample of semen is a sample of seminal plasma.
16. The method according to aspect 15, wherein the levels of sTWEAK, total PSA, FN14, sCD163 and glucose are determined.
17. The method according to any one of aspects 9 to 16, wherein the level of FN14 is the level of soluble FN14 (sFN14).
18. A kit comprising a reagent specific for determining the level of sTWEAK protein and at least an additional reagent specific for determining the level of a biomarker selected from the group consisting of total PSA, FN14, particularly sFN14, sCD163 and glucose.
19. The kit according to aspect 18, wherein said reagents are antibodies or fragments thereof, that specifically bind to said biomarkers.
20. Use of the kit according to any one of aspects 18 or 19 for:
   (a) the differential diagnosis of high risk or intermediate risk prostate cancer from low risk prostate cancer; or
   (b) for determining the likelihood that a patient with prostate cancer develops a high risk or intermediate risk prostate cancer; or
   (c) for selecting a therapy for a patient suffering from prostate cancer; or
   (d) for selecting a patient suffering from prostate cancer for a treatment selected from the group consisting of prostate surgery, radiotherapy and a combination thereof.

The invention is defined below by virtue of the following examples, which are to be construed as merely illustrative and not limitative of the scope of the invention.

### EXAMPLES

### MATERIALS AND METHODS

### Samples and patients

A total of n=84 between 18 and 75 year old patients with prostate cancer (PCa) who have undergone radical prostatectomy by open surgery were included. Inclusion criteria: Patients from 18 years onwards diagnosed of PCa by prostate biopsy in the inventor's centre or area of influence and treated by radical prostatectomy. Urine samples after prostate massage examination and semen was obtained from each patient. Exclusion criteria: the study excluded patients older than 75 year old with active treatment, recurrence or progression of any neoplastic process. Ethical and legal aspects: This proposal follows the principles of the Declaration of Helsinki, and has the approval of our CEIC at the time of its execution. All participants were asked their written informed consent prior to their inclusion. This project followed current legal regulations (Research Law Biomedical 14/2007; Royal Decree of Biobanks 1716/2011; Organic Law 15/1999 of September 13 Protection of Data of Personal Character). The study was approved by the Hospital/University Ethics-Committees and written informed consent from patients was obtained. All samples were obtained through the HJ23-Biobank.

Clinical parameters regarding tumour aggressiveness and metabolic status were collected. Patients with PCa were stratified into three risk groups following the criteria of D'amico 1: High risk (PSA> 20 ng / mL Gleason 8-10 T2c-T3), intermediate (PSA 10-20 ng / mL, Gleason 7, T2b) and low risk (PSA <10 ng / mL, Gleason 6, T1c, T2a) (A. V. D. Amico, et al., "Biochemical Outcome After Radical Prostatectomy , External Beam Radiation Therapy, or Interstitial Radiation Therapy for Clinically Localized Prostate Cancer," JAMA. 1998;280(11):969-974.

### Enzyme-Linked ImmunoSorbent Assay (ELISA) for TWEAK, Fn14 and CD163

Before performing the assay, the biological fluid samples were processed as follows: Urine: A volume of 3 mL of urine was centrifuged at 1500 g for 10 minutes at 4° C. The supernatant was collected for the study. Semen: Samples were centrifuged at 2000g for 15' at 22°C and the supernatant was collected for the study, which it is now called seminal plasma.

The quantification of the concentration of sTWEAK, sCD163 and sFn14 in the above mentioned biofluids was determined using ELISA sandwich in 96 well plates. Quantification of sTWEAK was performed by DuoSet TWEAK/TNFSF12 (R&D Systems; Minneapolis, MN, USA), sCD163 was performed by DuoSet CD163 (R&D Systems; Minneapolis, MN, USA) and for sFn14 the TNFSFR12A ELISA kit (Aviva Systems Biology, San Diego, California) was used. All ELISA Kits were used following the manufacturer's instructions, but changing the sample dilution as follows:

**Table 1 -Sample dilution (sample/simple diluent)**

| | **sTWEAK** | **sCD163** | **sFn14** |
|---|---|---|---|
| **Urine** | 3/1 | 3/1 | 3/1 |
| **Seminal plasma** | 1/10 | 1/5 | 1/5 |

### Standardization of results

The results obtained in each biofluid were normalised as follows: For Urine: levels of sTWEAK, sCD163 and sFn14 in urine were normalized with creatinine levels. In the case of seminal plasma samples, levels of sTWEAK, sCD163 and sFn14 were normalized with total protein levels in each sample. Quantification of protein levels was performed with the Pierce TM BCA Protein Assay Kit (Thermo Scientific, Rockford, Illinois, USA), following the bicinchoninic acid assay (BCA) method according to the manufacturer's instructions.

### Statistical analysis

The different individuals were grouped by D'Amico groups. All data were analyzed using IBM SPSS Statistics 19.0 for Windows (SPSS Inc., Chicago, IL, USA). The different parameters studied (sTWEAK, sCD163 and sFn14) were correlated in the different fluids (post-prostate stimulation urine and seminal plasma) with the proposed study groups. To compare between 2 groups Mann-Whitney's nonparametric test was used, since the data were not followed by a normal distribution. Precision classification and Receiver operating characteristic (ROC) curve analysis were conducted for clinical utility, sensitivity, specificity.

### RESULTS

### Levels of sTWEAK are reduced in seminal plasma of high risk PCa patients

Levels of sTWEAK, sFn14 and sCD163 were measured by ELISA in urine and seminal plasma samples of the cohort patients. Results showed that in urine samples levels of the above mentioned biomarkers are not useful to distinguish between high and low plus intermediate degrees of PCa patients stratified by D'Amico risk groups (**Figure 1**). Interestingly, in seminal plasma, the biomarker sTWEAK was the most useful for the identification of the aggressiveness of PCa, showing significant lower levels in patients with more aggressive PCa, while the other two examined biomarkers, sFn14 and sCD163 were partly reduced in the most aggressive cases but did not reach significance (**Figure 2**).

### Bivariate correlation analysis of biomarkers with clinical, pathological and metabolic markers

Spearman's correlation analysis showed that sTWEAK levels in seminal plasma significantly correlate positively with its receptor Fn14 levels **Figure 3A****.**

sTWEAK in seminal plasma also showed a clear negative correlation with total PSA levels **Figure 3B** and glucose levels **Figure 3C****.**

### Biomarker combination improves PCa diagnosis

In order to explore the potential value of circulating levels of sTWEAK in seminal plasma as a possible prognostic biomarker for PCa aggressiveness, a ROC analysis was performed to determine the weight exerted by the parameter sTWEAK on the risk of developing a high risk PCa. The Area under the Curve (AUC) of the ROC curve was 0.724 with a Confidence Interval (CI) of 95% between 0.58 and 0.87 (p = 0.008), that is, the probability that the diagnosis made to an aggressive case is more correct than that of a control chosen at random is 72.4%. Using this model, only 66.7% of patients can be classified with a sensitivity and specificity of 83.3% and 50%, respectively (**Figure 4A**).

Additionally, a ROC analysis for total PSA alone yielded an AUC of 0.779 with a CI of 95% between 0.65 and 0.91 (p = 0.001). Using this model, 69% of patients can be classified with a sensitivity and specificity of 83.7% and 57.1% respectively (**Figure 4A**). Then, a multivariate logistic regression (LR) analysis including both total PSA and sTWEAK levels in seminal plasma was performed. The resulting ROC curve had an AUC of 0.818 (IC of 95%=0.70-0.94; p<0.0001). Using this model, patient classification was improved up to 77.1% with a sensitivity and specificity of 83.3% and 62.5%, respectively (**Figure 4B**).

In order to improve the diagnosis, three more variables were introduced in a multivariate RL model: sFn14 levels in seminal plasma, sCD163 levels in seminal plasma and glucose plasma levels. sFn14 ROC curve had an AUC of 0.595 (IC of 95%=0.43-0.76; p=0.268); CD163 ROC curve had an AUC of 0.542 (IC of 95%=0.37-0.71; p=0.624); and Glucose ROC curve had an AUC of 0.578 (IC of 95%=0.41-0.75; p=0.359)(data not shown).

Finally, a multivariate LR model was proposed, being composed of 5 variables: total PSA serum levels, seminal levels of sTWEAK, seminal levels of sFn14, seminal levels of sCD163 and plasma glucose levels. The resulting ROC curve had an AUC of 0.854 (IC of 95%=0.74-0.97; P<0.0001), that is, inclusion of new variables improved the accuracy of the multivariate LR model composed only by total PSA and seminal sTWEAK. With this new model, **80%** of patients can be correctly classified with a sensitivity of **82.6%** and a specificity of **72.7%** (**Figure 4C**).

## Claims

1. An *in vitro* method for the differential diagnosis of high risk or intermediate risk prostate cancer from low risk prostate cancer, or for determining the likelihood that a patient with prostate cancer develops a high risk or intermediate risk prostate cancer, comprising:
(a) determining the level of soluble tumor necrosis factor ligand superfamily member 12 (sTWEAK) protein in a sample of semen from the patient and
(b) comparing said level with a reference value
wherein
- a decreased level of sTWEAK compared to the reference value is indicative that the patient suffers from a high risk or intermediate risk prostate cancer, or that there is a high likelihood that the patient develops a high risk or intermediate risk prostate cancer, or, alternatively,
- an equal or increased level of sTWEAK compared to the reference value is indicative that the patient suffers from low risk prostate cancer, or that there is a low likelihood that the patient develops a high risk or intermediate risk prostate cancer.

2. The method according to claim 1, further comprising
(a) determining the level of total prostate specific antigen (PSA) protein in a blood sample from said patient and
(b) comparing said level with a reference value,
wherein
- an increased level of total PSA compared to the reference value is indicative that the patient suffers from high risk or intermediate risk prostate cancer, or that there is a high likelihood that the patient develops a high risk or intermediate risk prostate cancer, or, alternatively,
- an equal or decreased level of total PSA compared to the reference value is indicative that the patient suffers from low risk prostate cancer, or that there is a low likelihood that the patient develops a high risk or intermediate risk prostate cancer.

3. The method according to claim 2, wherein the blood sample is a sample of serum.

4. The method according to any one of claims 1 to 3, further comprising:
(a) determining the level of at least one additional biomarker selected from the group consisting of: tumor necrosis factor receptor superfamily member 12A (FN14), particularly sFN14, soluble scavenger receptor cysteine-rich type 1 protein M130 (sCD163) and glucose, wherein the levels of FN14, particularly sFN14, and sCD163 are determined in a sample of semen and the level of glucose is determined in a blood sample and
(b) comparing said level with a reference value,
wherein
- a decreased level of FN14, particularly sFN14, and/or sCD163 compared to its reference value is indicative that the patient suffers from a high risk or intermediate risk prostate cancer, or that there is a high likelihood that the patient develops a high risk or intermediate risk prostate cancer, or, alternatively,
- an equal or increased level of FN14, particularly sFN14, and/or sCD163 compared to its reference value is indicative that the patient suffers from low risk prostate cancer, or that there is a low likelihood that the patient develops a high risk or intermediate risk prostate cancer.

5. The method according to claim 4, wherein the level of glucose is determined in a sample of plasma.

6. The method according to any one of claims 1 to 5, wherein the sample of semen is a sample of seminal plasma.

7. The method according to claim 6, wherein the levels of sTWEAK, total PSA, FN14, particularly sFN14, sCD163 and glucose are determined.

8. The method according to any one of claims 4 to 7, wherein the levels of FN14 are the levels of soluble FN14 (sFN14).

9. A method for selecting a therapy for a patient suffering from prostate cancer, comprising:
(a) determining the level of sTWEAK in a sample of semen from the patient and
(b) comparing said level with a reference value
wherein
- if a decreased level of sTWEAK compared to the reference value is detected, a therapy selected from the group consisting of prostate surgery, radiotherapy and a combination thereof is selected or, alternatively,
- if an equal or increased level of sTWEAK compared to the reference value is detected, an alternative therapy is selected.

10. A method for selecting a patient suffering from prostate cancer for a treatment selected from the group consisting of prostate surgery, radiotherapy and a combination thereof, comprising:
(a) determining the level of sTWEAK in a sample of semen from the patient and
(b) comparing said level with a reference value
wherein the patient is selected for said treatment if a decreased level of sTWEAK compared to the reference value is detected.

11. The method according to any one of claims 9 or 10, further comprising
(a) determining the level of total PSA in a blood sample from said patient and
(b) comparing said level with its reference value,
wherein
if an increased level of total PSA compared to its reference value is detected, a therapy selected from the group consisting of prostate surgery, radiotherapy and a combination thereof is selected, or the patient is selected for said treatment, or, alternatively,
if an equal or decreased level of total PSA compared to its reference value is detected, an alternative therapy is selected.

12. The method according to any one of claims 9 to 11, further comprising:
(a) determining the level of at least one additional biomarker selected from the group consisting of FN14, particularly sFN14, sCD163 and glucose, wherein the levels of FN14, particularly sFN14, and sCD163 are determined in a sample of semen and the level of glucose is determined in a blood sample and
(b) comparing said level with a reference value,
wherein
if a decreased level of FN14, particularly sFN14, and/or sCD163 compared to its reference value is detected, a therapy selected from the group consisting of prostate surgery, radiotherapy and a combination thereof is selected, or the patient is selected for said treatment, or, alternatively,
if an equal or increased level of FN14, particularly sFN14, and/or sCD163 compared to its reference value is detected, an alternative therapy is selected.

13. The method according to any one of claims 9 to 12, wherein the levels of sTWEAK, total PSA, FN14, particularly sFN14, sCD163 and glucose are determined.

14. A kit comprising a reagent specific for determining the level of sTWEAK protein and at least an additional reagent specific for determining the level of a biomarker selected from the group consisting of total PSA, FN14, particularly sFN14, sCD163 and glucose.

15. Use of the kit according to claim 14 for:
(a) the differential diagnosis of high risk or intermediate risk prostate cancer from low risk prostate cancer; or
(b) for determining the likelihood that a patient with prostate cancer develops a high risk or intermediate risk prostate cancer; or
(c) for selecting a therapy for a patient suffering from prostate cancer; or
(d) for selecting a patient suffering from prostate cancer for a treatment selected from the group consisting of prostate surgery, radiotherapy and a combination thereof.
